(19) Europäisches Patentamt
European Patent Office
Office européen des brevets

(11) EP 3 842 380 B1

(12) EUROPEAN PATENT SPECIFICATION

(45) Date of publication and mention of the grant of the patent:
**15.05.2024 Bulletin 2024/20**

(21) Application number: **20215667.5**

(22) Date of filing: **18.12.2020**

(51) International Patent Classification (IPC):
***B82Y 30/00*** *(2011.01)* ***G01N 30/34*** *(2006.01)*
***G01N 30/88*** *(2006.01)*

(52) Cooperative Patent Classification (CPC):
**B82Y 30/00;** G01N 30/34; G01N 2030/8822

(54) **A METHOD OF ANALYZING HEMOGLOBIN BY LIQUID CHROMATOGRAPHY, USE OF A CHROMATOGRAPHIC DEVICE FOR ANALYZING HEMOGLOBIN IN BLOOD AND USE OF A SURFACE TREATMENT LIQUID FOR TREATING THE FLOW PATH OF THE CHROMATOGRAPHIC DEVICE.**

VERFAHREN ZUR ANALYSE VON HÄMOGLOBIN DURCH FLÜSSIGCHROMATOGRAPHIE, VERWENDUNG EINER CHROMATOGRAPHISCHEN VORRICHTUNG ZUR ANALYSE VON HÄMOGLOBIN IN BLUT UND VERWENDUNG EINER OBERFLÄCHENBEHANDLUNGSFLÜSSIGKEIT ZUR BEHANDLUNG DES DURCHFLUSSWEGES DER CHROMATOGRAPHISCHEN VORRICHTUNG.

PROCEDÉE D'ANALYSE DE L'HÉMOGLOBINE PAR CHROMATOGRAPHIE LIQUIDE, L'UTILISATION D'UN DISPOSITIF CHROMATOGRAPHIQUE POUR ANALYSER L'HÉMOGLOBINE DANS LE SANG ET L'UTILISATION D'UN LIQUIDE DE TRAITEMENT DE SURFACE POUR TRAITER LA VOIE D'ÉCOULEMENT DU DISPOSITIF CHROMATOGRAPHIQUE.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.12.2019 JP 2019234558**
**01.12.2020 JP 2020199875**

(43) Date of publication of application:
**30.06.2021 Bulletin 2021/26**

(73) Proprietor: **ARKRAY, Inc.**
**Kyoto-shi, Kyoto 601-8045 (JP)**

(72) Inventor: **NAKAYAMA, Yusuke**
**Kyoto-shi, Kyoto 602-0008 (JP)**

(74) Representative: **Dehns**
**St. Bride's House**
**10 Salisbury Square**
**London EC4Y 8JD (GB)**

(56) References cited:
**EP-A1- 2 769 741**
**EP-A1- 3 450 972**
**FR-A- 2 831 180**
**US-A- 3 609 075**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 842 380 B1

**Description**

BACKGROUND

Technical Field

The present invention relates to a method of analyzing hemoglobin by liquid chromatography, to the use of a chromatographic device for analyzing hemoglobin in blood and to the use of a surface

treating liquid for
treating the flow
path of the chromatographic device.

Related Art

Usually, an analyzing device based on the principles of liquid chromatography is structured mainly of metal members of stainless steel, because high pressure resistance is required of the pipes and structural parts thereof. For example, as disclosed in Japanese Utility Model Application (JP-U) No. H06-7060, pipes, an injection valve of a sample, a housing of a column, and a metal filter provided at an introduction port of the column are metal parts. In particular, it is often the case that the filter is made of stainless steel as in Japanese Patent No. 2827649.

EP 3 450 972 A1 discloses a method of separating organic phosphor compounds by liquid chromatography. The method includes the use of a mobile phase comprising one or more solvents and an additive of pyrophosphoric acid or medronic acid.

Incidentally, as in Japanese Patent Application Laid-Open (JP-A) No. H06-331629, in order to accurately measure stable HbA1c as an index for diabetes, nitrilotris (methylenephosphonic acid) or the like is used as a dissociating agent that dissociates unstable HbAlc into HbA0 and glucose.

In a method for measuring hemoglobin in accordance with high-performance liquid chromatography, a phenomenon may arise whereby the widths of peaks detected in respective fractions of hemoglobin widen. If the width of a given peak widens, the peak coalesces with a subsequent peak, the border between the peaks becomes indistinct, and it is difficult to obtain good resolution. Namely, due to the width of a peak becoming larger, the peak overlaps the subsequent peak, and the border becomes unclear. For example, even within hemoglobin, as with HbA2 and HbS, the width of a peak of hemoglobin that is easily adsorbed to a column increases owing to a hydrophobic interaction between the hemoglobin and the column, or owing to an interaction caused by the charge of the hemoglobin and the charge of a carrier of the column, and adjacent peaks cannot be separated sufficiently.

SUMMARY

In a first aspect the method of the present invention relates to the analysis of hemoglobin in a blood sample by liquid chromatography. The method comprises treating the flow path of the liquid chromatography with a liquid in which is dissolved a surface treating agent having a molecular weight of 200 or less and having two or more chemical structures in each of which a hydroxy group (-OH) and an oxo group (=O) are respectively bonded directly to one phosphor (P) atom.

In accordance with the method of the present disclosure, the widths of hemoglobin peaks in a chromatogram obtained by liquid chromatography can be narrowed, and the resolution between the peaks improves.

BRIEF DESCRIPTION OF THE DRAWINGS

Exemplary embodiments will be described in detail based on the following figures, wherein:

Fig. 1 is a schematic drawing showing an overview of a high-performance liquid chromatography analysis system as an analyzing device at which a method of an embodiment of the present invention is implemented;
Fig. 2 is a hemoglobin analysis chart of an Example;
Fig. 3 is a hemoglobin analysis chart of a Comparative Example;
Fig. 4 is a virtual hemoglobin analysis chart for explaining a degree of separation of two peaks;
Fig. 5 shows a dissociation equilibrium reaction of methylene diphosphonate; and
Fig. 6 shows a dissociation equilibrium reaction of pyrophosphoric acid.

DETAILED DESCRIPTION

Because hemoglobin has a complex steric structure, an electric adsorption and a hydrophobic adsorption are strong, and it is easy for hemoglobin to bind to a surface of metal used in a pipe and a detector and the like of a measuring flow path, and to iron atoms in particular. At the time of analyzing hemoglobin, there are cases in which adjacent peaks coalesce and good resolution cannot be obtained. It is speculated that the reason for this is that, in a process in which a sample is introduced into an analyzing device and passes via a column and through the detector, the hemoglobin within a blood sample contacts and is adsorbed to members containing iron on the measuring flow path, and widths of detected peaks increase.

Because pressure resistance is required of the column, it is difficult to substitute metal with a resin, and, of necessity, a metal containing iron such as stainless steel is used. Further, at the pipe, although substitution with resins such as polyether ether ketone (PEEK) has advanced, substitution with a resin cannot be carried out within the detector, and portions at which iron atoms are exposed at the inner side surfaces exist.

Thus, in the method of the present embodiment, inner surfaces of a flow path of a liquid chromatography analyzing device are subjected to a surface treatment with a liquid (hereinafter also referred to as a surface treating liquid) in which a surface treating agent, which is a substance having a chemical structure described hereinafter, is dissolved. Thereby, binding of iron on the flow path and the hemoglobin is impeded, and the problem of the widths of the peaks increasing can thereby be resolved.

Namely, the surface treating agent has a chemical structure in which, within one molecule of the surface treating agent dissolved in a liquid, an ionized hydroxy group ($-O^-$) and an oxo group (=O) are respectively bonded directly to two or more phosphor atoms. In other words, the surface treating agent can also be called a compound in which hydroxy groups ($-O^-$), from which acidic protons have dissociated, and oxo groups (=O) are directly bonded to phosphor atoms, or, namely, can also be called an oxo acid. These two or more ionized hydroxy groups bind with metal atoms of the inner side surfaces of the flow path, and iron atoms in particular, and form a chelate. Namely, it can be thought that the hydroxy groups, which carry a negative charge and are bonded to different phosphor atoms within one molecule, bind owing to the electric interaction with the same one iron atom carrying a positive charge. Therefore, when a liquid, in which the surface treating agent is dissolved, soaks the flow path of a liquid chromatography analyzing device, the surface treating agent binds with the iron so as to cover the surfaces of the iron portions exposed at the inner side surfaces of the flow path. Further, it is thought that a compound, which binds to an iron atom by two hydroxy groups, is adsorbed more strongly to the iron atom than a compound binding to an iron atom via one hydroxy group. Therefore, it is difficult to replace the surface treating agent, which is in a state of being bound to the iron of the inner side surfaces of the flow path, with the hemoglobin flowing through the flow path, and it is difficult for the surface treating agent to separate from the inner side surfaces of the flow path. Therefore, it is possible to prevent the hemoglobin from directly contacting the metal. Further, the surface treating agent is a small molecule of a molecular weight of 200 or less, and a proportion of molecular weights of the hydroxy group and the oxo group, with respect to a molecular weight of the surface treating agent, is large. Because hydroxy groups and oxo groups are highly hydrophilic, the surface treating agent is highly hydrophilic. Accordingly, it is difficult for hemoglobin, which has strong hydrophobic adsorption, to adsorb to the surface treating agent covering the iron. Thus, it is thought that, by drenching the flow path, through which the sample flows, with the surface treating liquid, the widths of the peaks of the hemoglobin can be narrowed.

Here, in the above-described chemical structure of the surface treating agent, it is preferable that a second hydroxy group (-OH) binds to one phosphor (P) atom. Namely, the surface treating agent preferably has two or more chemical structures in each of which two hydroxy groups and one oxo group (=O) respectively are bonded directly to one phosphor atom. In this way, it is thought that, due to the surface treating agent having at least four hydroxy groups within one molecule, chelation with the iron atoms occurs at a higher efficiency and more frequently, and enlargement of the widths of the peaks can be more suppressed. Further, it is thought that, due to the surface treating agent having at least four hydroxy groups within one molecule, the hydrophilic property of the surface treating agent increases, and the hemoglobin is impeded to adsorb to the surface treating agent bonded to the metal portions of the flow path. Further, it is preferable that the surface treating agent does not include a functional group that is highly hydrophobic, such as a phenyl group or an alkyl group for example. By using a surface treating agent that does not contain a highly hydrophobic functional group, it is difficult for hemoglobin, which has strong hydrophobic adsorption, to be adsorbed to the surface treating agent covering the iron. Further, it is preferable that the surface treating agent does not contain a functional group having an acid dissociation constant lower than that of a hydroxy group, such as a carboxy group or a sulfo group for example. By using a surface treating agent that does not contain a functional group having a lower acid dissociation constant than a hydroxy group, it is difficult for hemoglobin, which has strong electric adsorption, to be adsorbed to the surface treating agent covering the iron. Methylene diphosphonate (molecular weight 176) expressed by the structural formula shown by following chemical formula 1, or 1,2-ethylene diphosphonate (molecular weight 190.03), or pyrophosphoric acid (molecular weight 177.98) expressed by the structural formula shown by following chemical formula 2, may be used as this surface treating agent. Among these, methylene diphosphonate is the most preferable as the surface

treating agent. Note that a type of an atom that joins the two or more phosphor (P) atoms that the surface treating agent possesses is not limited, and may be, for example, carbon (C) or nitrogen (N) or oxygen (O), or the two or more phosphor (P) atoms may be joined via plural types of atoms. Further, a number of atoms that join the two or more phosphor (P) atoms that the surface treating agent has also is not limited.

] [Chemical Formula 1]

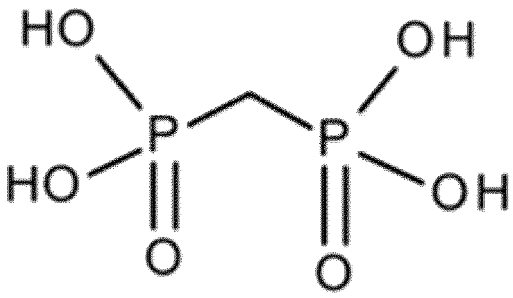

[Chemical Formula 2]

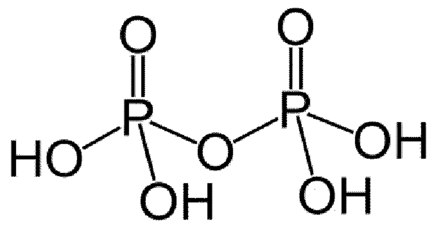

The flow path subjected to surface treatment with the surface treating liquid is the flow path through which the sample flows. Specifically, this is the flow path over which the sample travels via the column until reaching the hemoglobin detecting section. For example, it suffices to carry out a surface treatment by using the surface treating liquid on the flow path connecting the injector introduces the sample into the column and the column provided downstream of the injector, and on the flow path connecting the column and the detecting section provided downstream of the column.

In the surface treatment of the flow path, it suffices to make the surface treating liquid contact the inner surfaces of the flow path. For example, an interior of the flow path may be immersed in the surface treating liquid, or the surface treating liquid may be made to flow through the flow path. Although it is preferable for all of the inner surfaces of the flow path to be subjected to the surface treatment with the surface treating liquid, in a case in which the portions of the flow path at which metal is exposed are known, the surface treatment may be carried out on only those portions. For example, in a case in which, of the flow path, metal is exposed at the inner surfaces of the column, the surface treatment may be carried out on the inner surfaces of the column only. Further, although it is preferable that, of the inner surfaces of the flow path, the surface treatment be carried out on all of the portions at which metal is exposed, the surface treatment may be carried out on only some of the portions at which metal is exposed.

Here, the surface treatment of the flow path by the surface treating liquid may be carried out by immersing the flow path in a container filled with the surface treating liquid, or may be carried out by immersing the parts constituting the flow path in a container filled with the surface treating liquid, and assembling the flow path by using these parts. Further, the inner surfaces may be immersed by causing the surface treating liquid to flow through the flow path provided in a liquid chromatography analyzing device. In this case, the surface treating agent may be contained in at least one of an eluent, a washing liquid, or a dilution liquid for the sample, which are used in the hemoglobin analysis, and this liquid containing the surface treating agent may be made to flow through the flow path. Or, the surface treating liquid, which is readied separately from the eluent, may be made to flow through the flow path. The surface treating liquid may be delivered into the flow path by using a pump provided at the liquid chromatography analyzing device, or by using another pump.

Note that, even if the inner surfaces of the flow path are once subjected to a surface treatment by using the surface treating liquid, it is possible that the effect of the surface treatment will be damped by repeating hemoglobin analysis thereafter. Therefore, the flow path may be subjected to a surface treatment before hemoglobin analysis. For

example, every time when hemoglobin analysis is completed, a surface treating liquid in which the surface treating agent is dissolved may be made to flow through the flow path. Further, by carrying out hemoglobin analysis by using an eluent in which the surface treating agent is dissolved, the surface treatment of the flow path may be carried out simultaneously with the separation analysis of the hemoglobin. Further, by diluting the sample in a dilution liquid containing the surface treating agent, and introducing this diluted sample into the flow path and carrying out hemoglobin analysis thereon, the surface treatment of the flow path may be carried out simultaneously with the separation analysis of the hemoglobin.

It suffices for the liquid in which the surface treating agent is dissolved to be a liquid that can ionize the hydroxy groups of the surface treating agent. For example, the liquid may be water, or may be a buffer solution in which a pH buffering agent is dissolved in water. Or, the liquid may be an eluent used in liquid chromatography, or may be a dilution liquid for the sample. The liquid may be a washing liquid washing the adsorped components off from the carrier of the column by being delivered to the column.

The pH of the liquid in which the surface treating agent is dissolved is preferably a pH by which the surface treating agent becomes a structure having two or more chemical structures in each of which an acidic-proton-dissociated hydroxy group is bonded to one phosphor (P) atom. More specifically, the pH of the liquid in which the surface treating agent is dissolved preferably has a higher pH than the acid dissociation constant pKa at the time when the surface treating agent becomes a structure having two chemical structures in each of which an acidic-proton-dissociated hydroxy group is bonded to one phosphor (P) atom, as a result of a dissociation of acidic protons from the hydroxy group. Here, a configuration, which has a chemical structure in which an acidic-proton-dissociated hydroxy group and an oxo group are respectively bonded directly to one phosphor (P) atom and a chemical structure in which a non-dissociated hydroxy group and an oxo group are respectively bonded directly to one phosphor (P) atom, is defined as a "first configuration". Further, a configuration in which an acidic proton has been dissociated from the non-dissociated hydroxy group in the first configuration is defined as a "second configuration". That is, the second configuration has two chemical structures in each of which an acidic-proton-dissociated hydroxy group and an oxo group are respectively bonded directly to one phosphor (P) atom. In other words, it is preferable that the pH of the liquid in which the surface treating agent is dissolved is larger than an acid dissociation constant pKa in an equilibrium reaction between the first configuration and the second configuration. Namely, due to the pH of the surface treating liquid being a higher value than this pKa, the surface treating agent, which has two chemical structures in each of which an acidic-proton-dissociated hydroxy group is bonded to one phosphor (P) atom, accounts for over half of the surface treating agent dissolved in the liquid, and therefore, the aforementioned chelation with iron atoms occurs at a higher frequency. Further, the number of hydroxy groups that are ionized among the hydroxy groups within one molecule of the surface treating agent is larger, and the surface treating agent can bind more strongly to the iron.

Following Table 1 lists acid dissociation constants $pKa_1$ to $pKa_4$ at the time when one to four acidic protons dissociate, among the four hydroxy groups existing within one molecule, respectively for methylene diphosphonate and pyrophosphoric acid as the surface treating agent. The acid dissociation constant $pKa_2$, by which the surface treating agent becomes a structure having two chemical structures in each of which one dissociated hydroxy group and one non-dissociated hydroxy group are bonded to one phosphor (P) atom as a result of the dissociation of acidic protons from methylene diphosphonate is 3.05. The acid dissociation constant $pKa_3$ at the time when one more acidic proton dissociates is 7.35, and the acid dissociation constant $pKa_4$ at the time when yet one more acidic proton dissociates is 10.96. Similarly, the acid dissociation constant $pKa_2$, by which the surface treating agent becomes a structure having two chemical structures in each of which one dissociated hydroxy group and one non-dissociated hydroxy group are bonded to one phosphor (P) atom as a result of the dissociation of acidic protons from pyrophosphoric acid is 2.0. The acid dissociation constant $pKa_3$ at the time when one more acidic proton dissociates is 6.6, and the acid dissociation constant $pKa_4$ at the time when yet one more acidic proton dissociates is 9.4. Therefore, regardless of the type of surface treating agent, an effective pH of the surface treating liquid is 3.05 or more, and preferably 7.35 or more, and more preferably 10.96 or more.

[Table 1]

| surface treating agent | acid dissociation constant | | | |
|---|---|---|---|---|
| | $pKa_1$ | $pKa_2$ | $pKa_3$ | $pKa_4$ |
| methylene diphosphonate | 1.43 | 3.05 | 7.35 | 10.96 |
| pyrophosphoric acid | 0.9 | 2.0 | 6.6 | 9.4 |

Although the concentration of the surface treating agent contained in the surface treating liquid does not matter, it is 5 $\mu$M or more, and preferably 10 $\mu$M or more.

EXAMPLES

An example in which a flow path was subjected to a surface treatment with a surface treating liquid by causing a washing liquid, in which the surface treating agent was dissolved, to flow through the flow path before separation analysis of hemoglobin, is shown as follows. Note that, in a case in which the flow path is washed by causing a washing liquid, in which the surface treating agent is dissolved, to flow through the flow path after the separation analysis, the flow path has already been subjected to the surface treatment with the surface treating agent as a result of this washing. Therefore, it is not necessary to deliver the washing liquid, in which the surface treating agent is dissolved, to the flow path before carrying out the next separation analysis. Further, because the method of the present disclosure can be applied to analysis based on the principles of liquid chromatography, the present disclosure is not limited to the HPLC analysis system as described in the following Example.

The method of the present disclosure was implemented at an analyzing device 10 as a high-performance liquid chromatography (HPLC) analyzing system as illustrated in Fig. 1. In this analyzing device 10, flow paths 40, 50 were connected respectively to an upstream side and a downstream side of an HPLC column 20 filled with a filler 21 that was a carrier suitable for hemoglobin that was the subject of analysis. An injector 60 introducing liquid into the flow path 40 was provided further upstream of the upstream-side flow path 40. A storage tank 61 storing the liquid was positioned at the upstream-most side of the injector 60. A pump 62 delivering the liquid stored in the storage tank 61 toward the downstream side was positioned downstream of the storage tank 61. An introducing section 63, which merged a pretreating agent flow path 41 introducing a pretreating agent and a sample flow path 42 introducing the blood sample, was positioned at the downstream side of the pump 62 on the flow path 40 running from the pump 62 to the HPLC column 20.

The storage tank 61 was sectioned into plural regions, and two types of eluents, and a washing liquid in which the surface treating agent was dissolved, were stored in these respective regions so as not to mix with one another. The pump 62 was able to mix the two types of eluents, which were stored in the storage tank, together in an arbitrary proportion, to deliver the mixed liquid toward the downstream side, and separately therefrom, and also to deliver the washing liquid toward the downstream side. A light source 71 illuminating light (shown by an arrow in Fig. 1) including an absorption wavelength of hemoglobin into the flow path 50, and a detector 72, which received the light illuminated from the light source 71 and passed through the interior of the flow path 50, were provided at the flow path 50 at the downstream side of the HPLC column 20. The portion in the flow path 50, at which the hemoglobin was detected by the light source 71 and the detector 72, was a detecting section 70.

In the present Example, the blood sample, which was introduced in the introducing section 63 of the injector 60 via the sample flow path 42, flowed through the flow path 40 at the upstream side, the HPLC column 20, and the flow path 50, in this order. Here, metal was exposed at inner surfaces of the flow path 50 connecting the HPLC column 20 and the detecting section 70. Therefore, if the flow path 50 through which the blood sample flowed had not been subjected to a surface treatment with the surface treating agent, hemoglobin would have contacted and been adsorbed to the metal in the process of the hemoglobin contained in the blood sample reaching the detector 72.

The washing liquid in the present Example had the composition shown in following Table 2. Note that the pH of this washing liquid was adjusted appropriately to 6.85 higher than 3.05 (see above Table 1) that was the $pKa_2$ of methylene diphosphonate as the surface treating agent.

[Table 2]

| component | concentration (g/L) | molecular weight | mol concentration (mM) |
|---|---|---|---|
| citric acid monohydrate | 1.1 | 210.14 | 5.235 |
| disodium hydrogen phosphate | 4.36 | 141.96 | 30.71 |
| disodium EDTA | 0.1 | 292.24 | 0.3422 |
| methylene diphosphonate | 0.002 | 176 | 0.01136 |

A first liquid as an eluent in the present Example had the composition shown in following Table 3. Note that the pH of this first liquid was adjusted appropriately to 5.25.

[Table 3]

| component | concentration (g/L) | molecular weight | mol concentration (mM) |
|---|---|---|---|
| sodium dihydrogen phosphate | 14 | 119.98 | 116.7 |
| disodium hydrogen phosphate | 0.35 | 141.96 | 2.465 |

(continued)

| component | concentration (g/L) | molecular weight | mol concentration (mM) |
|---|---|---|---|
| sodium propionate | 0.1 | 96.07 | 1.041 |
| disodium EDTA | 0.1 | 292.24 | 0.3422 |

A second liquid as an eluent in the present Example had the composition shown in following Table 4. Note that the pH of this second liquid was adjusted appropriately to 6.85. Note that the composition of the washing liquid and the composition of the second liquid differed only with respect to whether or not methylene diphosphonate was included therein. Therefore, the washing liquid may be used as the second liquid as an eluent. In such a case, the first liquid, the second liquid and the washing liquid do not have to be prepared separately, and it suffices to prepare only the washing liquid and the first liquid.

[Table 4]

| component | concentration (g/L) | molecular weight | mol concentration (mM) |
|---|---|---|---|
| citric acid monohydrate | 1.1 | 210.14 | 5.235 |
| disodium hydrogen | 4.36 | 141.96 | 30.71 |
| phosphate | | | |
| disodium EDTA | 0.1 | 292.24 | 0.3422 |

In a Comparative Example for comparison with the above-described Example, a liquid of a composition that, among the components of the composition shown in Table 1, did not contain methylene diphosphonate was used as the washing liquid, and the pH thereof was appropriately adjusted to 6.85. The composition of the first liquid, the composition of the second liquid, and the analyzing device 10 were the same as those of the above-described Example.

In the above-described Example and Comparative Example, first, the washing liquid was delivered from the storage tank 61 to the flow paths 40, 50 by using the pump 62. Thereby, in the Example, the inner surfaces of the flow paths 40, 50 were immersed by a liquid containing methylene diphosphonate as the surface treating agent, and were subjected to the surface treatment. On the other hand, because the washing liquid of the Comparative Example did not contain methylene diphosphonate, the inner surfaces of the flow paths 40, 50 were not subjected to a surface treatment with a liquid containing the surface treating agent.

Thereafter, after the first liquid was delivered out into the flow path 40, a blood sample was introduced from the sample flow path 42 into the introducing section 63 within the injector 60. Then, the first liquid, and a mixed liquid of a pH of 6.4 in which the first liquid and the second liquid were mixed together at a ratio of 3:7, and a mixed liquid of a pH of 6.7 in which the first liquid and the second liquid were mixed together at a ratio of 1:9, were successively delivered into the flow path 40. The blood sample, which was introduced into the flow path 40 together with the eluent, was delivered to the HPLC column 20, and the hemoglobin contained in the blood sample was divided into respective hemoglobin sections thereat. The light absorbances of the divided hemoglobin sections were measured by the detector 72 provided at the flow path 50 at the downstream side of the HPLC column 20. Then, a chromatogram, which expressed the light absorbances with respect to time elapsed from the time when the blood sample was introduced into the flow path 40, was obtained.

An HbA peak, an HbA2 peak and an HbS peak at the time when hemoglobin was analyzed under the conditions of the above-described Example are respectively shown in a chart of Fig. 2. Further, an HbA peak, an HbA2 peak and an HbS peak at the time when hemoglobin was analyzed under the conditions of the above-described Comparative Example are respectively shown in the chart of Fig. 3. Note that, in each of these drawings, the vertex of the HbA peak is omitted. As can be understood from each of these drawings, a distal end of the HbA2 peak is more narrow in the Example than in the Comparative Example, and the width of the peak is also more narrow. This trend is the same at the HbA peak and the HbS peak adjacent to the HbA2 peak. From this, it is presumed that widening of the widths of the peaks was suppressed by the surface treatment of the flow path 50 by methylene diphosphonate as the surface treating agent.

Here, two peaks as objects of analysis are supposed as shown in the virtual hemoglobin analysis chart shown in Fig. 4. A vertical axis and a horizontal axis in a chart of Fig. 4 are, in the same way as Fig. 2 and Fig. 3, light absorbance and elapsed time (seconds). The time corresponding to a vertex of a peak at the left side in the drawing (hereinafter called "first peak") is $t_{R1}$, and the time corresponding to a vertex of a peak at the right side in the drawing (hereinafter called "second peak") is $t_{R2}$. Further, given that the height of the first peak is hi, and the height of the second peak is

$h_2$, the width of the peak at the height of $0.5h_1$ in the first peak (hereinafter called "half-value width" (whose unit is time (seconds)) is $W_{0.5h1}$, and the half value width at the height of $0.5h_2$ in the second peak is $W_{0.5h2}$. At this time, a degree of separation R between the first peak and the second peak is defined by the following numerical formula.

[Numerical Formula 1]

$$R = 1.18 \times \left( \frac{t_{R2} - t_{R1}}{W_{0.5h1} + W_{0.5h2}} \right)$$

Namely, the higher the distance between the peaks (i.e., the time difference at which the peaks arise) defined as $t_{R2}$-$t_{R1}$ in the above formula, and, the smaller the width of each peak, the higher the degree of separation between the two peaks.

Here, the half value width ($\alpha_1$) of the HbA peak, the half value width ($\beta_1$) of the HbA2 peak, and the inter-peak distance ($\delta_1$) in the Example shown in Fig. 2, and the half value width ($\alpha_2$) of the HbA peak, the half value width ($\beta_2$) of the HbA2 peak, and the inter-peak distance ($\delta_2$) in the Comparative Example shown in Fig. 3, were as in following Table 5, and the respective separation degrees (R) thereof also were as listed in Table 5.

[Table 5]

| Example/ Comp. Ex. | half value width (sec) | | inter-peak distance (sec) | degree of separation |
|---|---|---|---|---|
| | HbA | HbA2 | | |
| Example | 0.6 | 0.2 | 1.5 | 2.21 |
| Comp. Ex. | 0.8 | 0.8 | 1.7 | 1.25 |

Further, the half value width ($\beta_1$) of the HbA2 peak, the half value width ($\gamma_1$) of the HbS peak and the inter-peak distance ($\varepsilon_1$) in the Example shown in Fig. 2, and the half value width ($\beta_2$) of the HbA2 peak, the half value width ($\gamma_2$) of the HbS peak and the inter-peak distance ($\varepsilon_2$) in the Comparative Example shown in Fig. 3, were as in following Table 6, and the respective separation degrees (R) thereof also were as listed in Table 6.

[Table 6]

| Example/ Comp.Ex. | half value width (sec) | | inter-peak distance (sec) | degree of separation |
|---|---|---|---|---|
| | HbA2 | HbS | | |
| Example | 0.2 | 0.7 | 1.9 | 2.49 |
| Comp. Ex. | 0.8 | 1.2 | 4.0 | 2.36 |

With respect to each of the HbA peak, the HbA2 peak and HbS peak, as shown in above Table 5 and Table 6, it was shown that the half value width in the Example was shorter than that in the Comparative Example, and the width of the peak in the Example was narrower than that of the Comparative Example.

On the other hand, as to the inter-peak distances between the HbA peak and the HbA2 peak, and the inter-peak distances between the HbA2 peak and the HbS peak, the Comparative Example showed longer distances than the Example. It is assumed that this was caused by the broad peak widths in the Comparative Example.

Further, as to the degrees of separation between the HbA peak and the HbA2 peak, and the degrees of separation between the HbA2 peak and the HbS peak, it was resulted that the Example showed higher degrees than the Comparative Example. It is thought that the result was caused by the fact that the methylene diphosphonate as the surface treating agent was adsorbed to the iron of the inner surfaces of the flow path 50, and that the hemoglobin was prevented from being adsorbed to the metal. Further, it is shown that, by delivering the washing liquid containing the methylene diphosphonate before the separation analysis, the resolution between the peaks can be improved even if methylene diphosphonate is not contained in the eluent used in the separation analysis thereafter. It is thought that this

is caused by the surface treating agent being strongly adsorbed to the iron of the inner surfaces of the flow path because the ionized hydroxy groups, which bind to different phosphor atoms within one molecule of the methylene diphosphonate, bind to the same single iron atom.

As described above, it is shown that, in the Example, by adding methylene diphosphonate as a surface treating agent to a washing liquid, the widths of the peaks of hemoglobin were prevented from widening, and the resolution between peaks was improved.

Industrial Applicability

The present invention can be used in hemoglobin analysis in accordance with high-performance liquid chromatography.

## Claims

1. A method for analyzing hemoglobin in a blood sample by liquid chromatography comprising subjecting the flow path (40,50) of the liquid chromatography to a surface treatment with a liquid in which is dissolved a surface treating agent having a molecular weight of 200 or less and having two or more chemical structures in each of which a hydroxy group (-OH) and an oxo group (=O) are respectively bonded directly to one phosphorus (P) atom, wherein the surface treatment is carried out before, or simultaneously with, hemoglobin analysis of the blood sample and wherein the surface treatment agent is bound to metallic portions of the flow path.

2. The method of claim 1, wherein, in each of the chemical structures of the surface treating agent, a second hydroxy group (-OH) is bonded to the one phosphorus (P) atom.

3. The method of claim 1 or claim 2, wherein the surface treating agent comprises methylene diphosphonate.

4. The method of any one of claim 1 to claim 3, wherein the pH of the liquid in which the surface treating agent is dissolved is a higher value than the pKa at which the surface treating agent becomes a structure having two chemical structures in each of which an acidic-proton-dissociated hydroxy group ($-O^-$) and an oxy group (=O) are respectively bonded directly to one phosphorus (P) atom, as a result of a dissociation of acidic protons from the surface treating agent.

5. Use of a chromatographic device (10) in the analysis of hemoglobin within a blood sample by liquid chromatography, the device comprising:

   a flow path (40, 50) through which the blood sample and an eluent flow,
   wherein a surface treating agent, having a molecular weight of 200 or less and having two or more chemical structures in each of which a hydroxy group (-OH) and an oxo group (=O) are respectively bonded directly to one phosphorus (P) atom, is bonded to metallic portions of the flow path.

6. Use of a surface treating liquid for treating metallic portions of a flow path (40, 50) in the liquid chromatography analysis of hemoglobin in a blood sample, the surface treating liquid comprising a surface treating agent having a molecular weight of 200 or less and having two or more chemical structures in each of which a hydroxy group (-OH) and an oxo group (=O) are respectively bonded directly to one phosphorus (P) atom wherein the surface treatment agent is bound to metallic portions of the flow path.

7. The use of claim 6, wherein the surface treating liquid is an eluent.

8. The use of claim 6, wherein the surface treating liquid is a washing liquid.

9. The use of claim 6, wherein the surface treating liquid is a dilution liquid for the blood sample.

## Patentansprüche

1. Verfahren zum Analysieren von Hämoglobin in einer Blutprobe mittels Flüssigkeitschromatographie, umfassend Unterziehen des Strömungswegs (40, 50) der Flüssigkeitschromatographie einer Oberflächenbehandlung mit einer

Flüssigkeit, in welcher ein Oberflächenbehandlungsmittel gelöst ist, das ein Molekulargewicht von 200 oder weniger aufweist, und zwei oder mehr chemische Strukturen aufweist, in welchen jeweils eine Hydroxygruppe (-OH) und eine Oxogruppe (=O) jeweils direkt an ein Phosphoratom (P) gebunden sind, wobei die Oberflächenbehandlung vor oder gleichzeitig mit der Hämoglobinanalyse der Blutprobe durchgeführt wird, und wobei das Oberflächenbehandlungsmittel an metallische Abschnitte des Strömungswegs gebunden ist.

2. Verfahren nach Anspruch 1, wobei in jeder der chemischen Strukturen des Oberflächenbehandlungsmittels eine zweite Hydroxygruppe (-OH) an das eine Phosphoratom (P) gebunden ist.

3. Verfahren nach Anspruch 1 oder Anspruch 2, wobei das Oberflächenbehandlungsmittel Methylendiphosphonat umfasst.

4. Verfahren nach einem von Anspruch 1 bis Anspruch 3, wobei der pH-Wert der Flüssigkeit, in welcher das Oberflächenbehandlungsmittel gelöst ist, ein höherer Wert ist als der pKa-Wert, bei welchem das Oberflächenbehandlungsmittel zu einer Struktur wird, die zwei chemische Strukturen aufweist, in welchen jeweils eine vom sauren Proton dissoziierte Hydroxygruppe (-O) und eine Oxygruppe (=O) jeweils infolge einer Dissoziation saurer Protonen aus dem Oberflächenbehandlungsmittel direkt an ein Phosphoratom (P) gebunden sind.

5. Verwendung einer chromatographischen Vorrichtung (10) bei der Analyse von Hämoglobin in einer Blutprobe durch Flüssigkeitschromatographie, wobei die Vorrichtung umfasst:

einen Strömungsweg (40, 50), durch welchen die Blutprobe und ein Elutionsmittel strömen,
wobei ein Oberflächenbehandlungsmittel, das ein Molekulargewicht von 200 oder weniger aufweist, und zwei oder mehr chemische Strukturen aufweist, in welchen jeweils eine Hydroxygruppe (-OH) und eine Oxogruppe (=O) jeweils direkt an ein Phosphoratom (P) gebunden sind, an metallische Abschnitte des Strömungswegs gebunden ist.

6. Verwendung einer Oberflächenbehandlungsflüssigkeit zum Behandeln metallischer Abschnitte eines Strömungswegs (40, 50) bei der Flüssigkeitschromatographie-Analyse von Hämoglobin in einer Blutprobe, wobei die Oberflächenbehandlungsflüssigkeit ein Oberflächenbehandlungsmittel umfasst, das ein Molekulargewicht von 200 oder weniger aufweist, und zwei oder mehr chemische Strukturen aufweist, in welchen jeweils eine Hydroxygruppe (-OH) und eine Oxogruppe (=O) jeweils direkt an ein Phosphoratom (P) gebunden sind, wobei das Oberflächenbehandlungsmittel an metallische Abschnitte des Strömungswegs gebunden ist.

7. Verwendung nach Anspruch 6, wobei die Oberflächenbehandlungsflüssigkeit ein Elutionsmittel ist.

8. Verwendung nach Anspruch 6, wobei die Oberflächenbehandlungsflüssigkeit eine Waschflüssigkeit ist.

9. Verwendung nach Anspruch 6, wobei die Oberflächenbehandlungsflüssigkeit eine Verdünnungsflüssigkeit für die Blutprobe ist.

## Revendications

1. Procédé d’analyse de l’hémoglobine dans un échantillon de sang par chromatographie en phase liquide comprenant la soumission du trajet d’écoulement (40, 50) de la chromatographie en phase liquide à un traitement de surface avec un liquide dans lequel est dissous un agent de traitement de surface présentant un poids moléculaire de 200 ou moins et présentant deux, ou plus, structures chimiques dans chacune desquelles un groupe hydroxy (-OH) et un groupe oxo (=O) sont respectivement liés directement à un atome de phosphore (P), dans lequel le traitement de surface est effectué avant, ou en même temps que, l’analyse de l’hémoglobine de l’échantillon de sang, et dans lequel l’agent de traitement de surface est lié à des parties métalliques du trajet d’écoulement.

2. Procédé selon la revendication 1, dans lequel, dans chacune des structures chimiques de l’agent de traitement de surface, un second groupe hydroxy (-OH) est lié à l’atome de phosphore (P).

3. Procédé selon la revendication 1 ou la revendication 2, dans lequel l’agent de traitement de surface comprend du diphosphonate de méthylène.

4. Procédé selon l'une quelconque de la revendication 1 à la revendication 3, dans lequel le pH du liquide dans lequel l'agent de traitement de surface est dissous, est une valeur supérieure au pKa auquel l'agent de traitement de surface devient une structure présentant deux structures chimiques dans chacune desquelles un groupe hydroxy dissocié par un proton acide (-O ) et un groupe oxy (=O) sont respectivement liés directement à un atome de phosphore (P), suite à une dissociation de protons acides à partir de l'agent de traitement de surface.

5. Utilisation d'un dispositif chromatographique(10) dans l'analyse de l'hémoglobine dans un échantillon de sang par une chromatographie en phase liquide, le dispositif comprenant :

   un trajet d'écoulement (40, 50) à travers lequel s'écoulent l'échantillon de sang et un éluant,
   dans lequel un agent de traitement de surface, présentant un poids moléculaire de 200 ou moins et présentant deux, ou plus, structures chimiques dans chacune desquelles un groupe hydroxy (-OH) et un groupe oxo (=O) sont respectivement liés directement à un atome de phosphore (P), est lié à des parties métalliques du trajet d'écoulement.

6. Utilisation d'un liquide de traitement de surface pour traiter des parties métalliques d'un trajet d'écoulement (40, 50) dans l'analyse par chromatographie en phase liquide de l'hémoglobine dans un échantillon de sang, le liquide de traitement de surface présentant un agent de traitement de surface présentant un poids moléculaire de 200 ou moins et présentant deux, ou plus, structures chimiques dans chacune desquelles un groupe hydroxy (-OH) et un groupe oxo (=O) sont respectivement liés directement à un certain atome de phosphore (P), dans laquelle l'agent de traitement de surface est lié à des parties métalliques du trajet d'écoulement.

7. Utilisation selon la revendication 6, dans laquelle le liquide de traitement de surface est un éluant.

8. Utilisation selon la revendication 6, dans laquelle le liquide de traitement de surface est un liquide de lavage.

9. Utilisation selon la revendication 6, dans laquelle le liquide de traitement de surface est un liquide de dilution pour l'échantillon de sang.

FIG.1

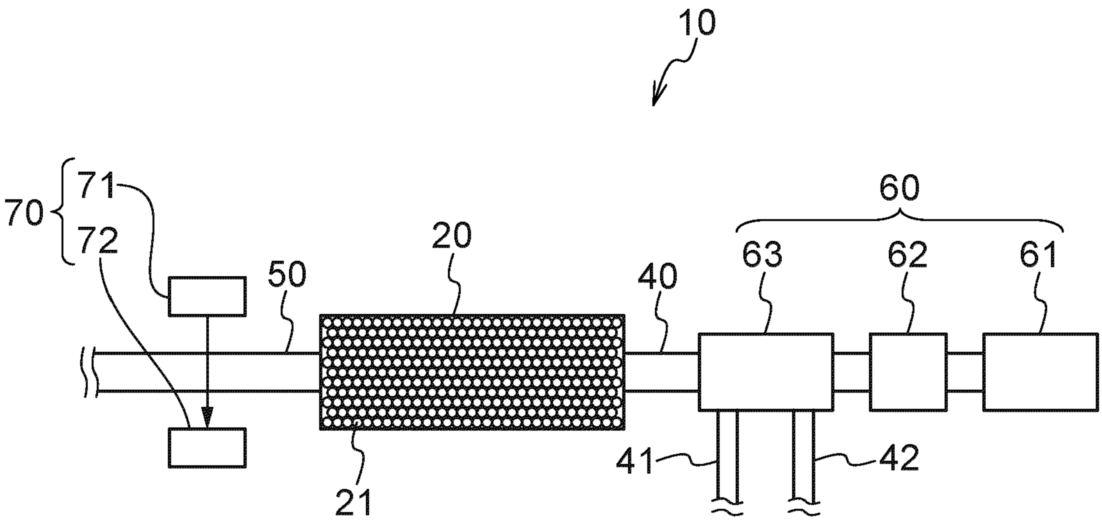

FIG.2

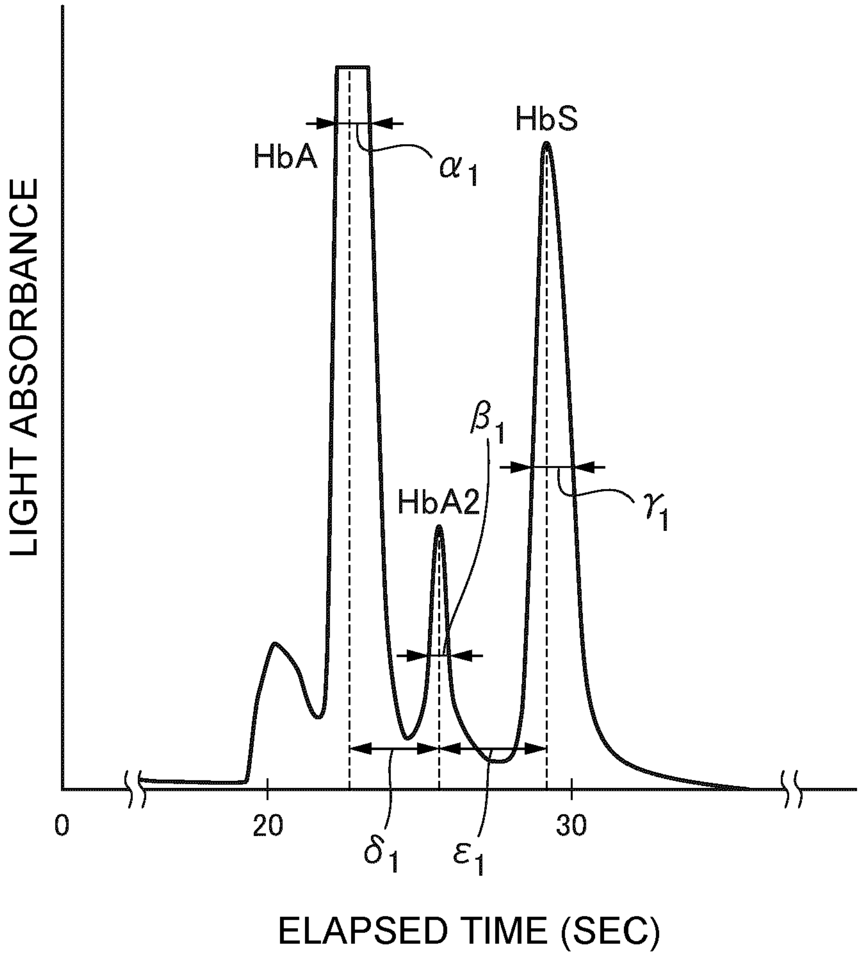
LIGHT ABSORBANCE
HbA
α1
HbS
β1
HbA2
γ1
0
20
30
δ1
ε1
ELAPSED TIME (SEC)

FIG.3

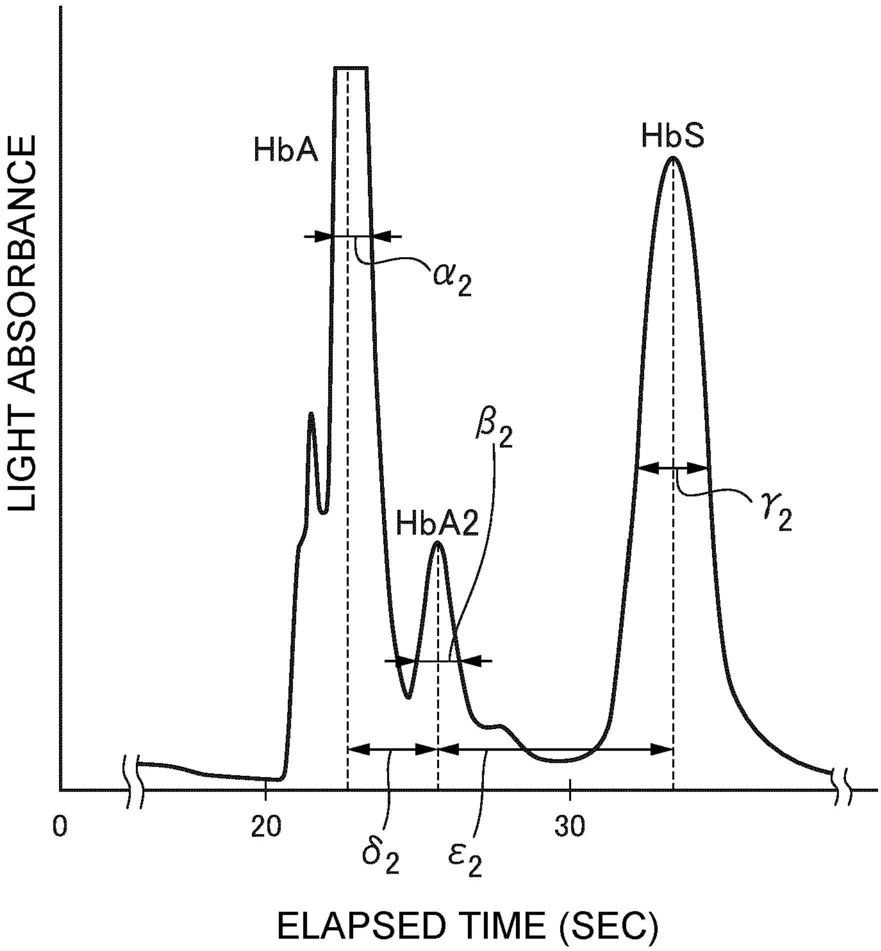
LIGHT ABSORBANCE
HbA
HbS
α2
β2
HbA2
γ2
0
20
30
δ2
ε2
ELAPSED TIME (SEC)

FIG.4

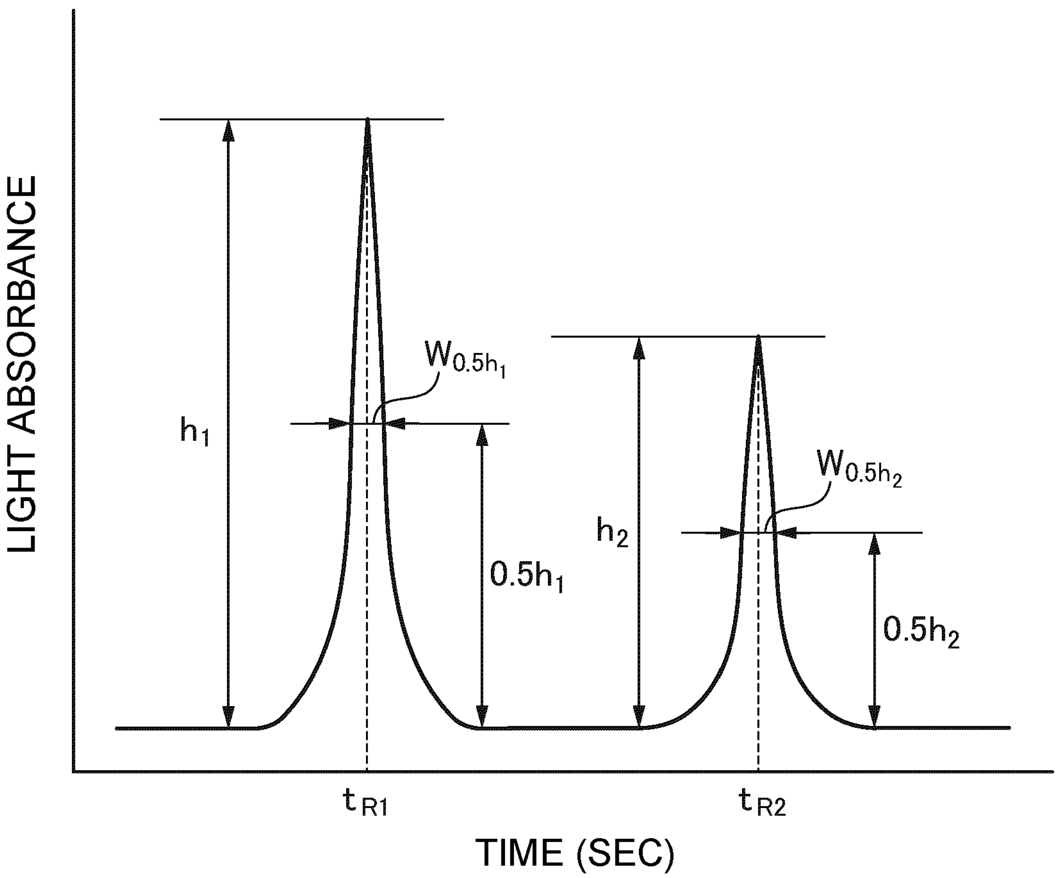
LIGHT ABSORBANCE
h1
W0.5h1
0.5h1
h2
W0.5h2
0.5h2
tR1
tR2
TIME (SEC)

FIG.5

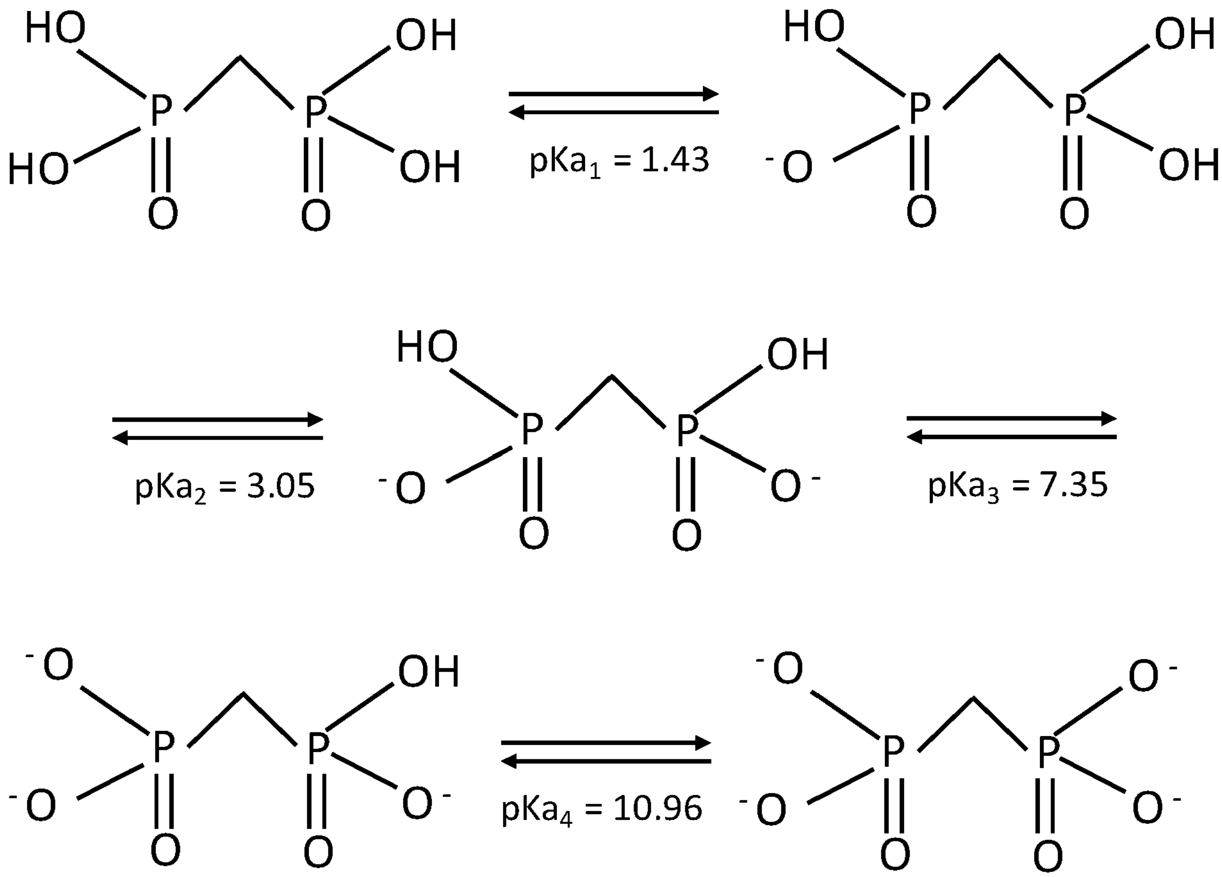

FIG.6

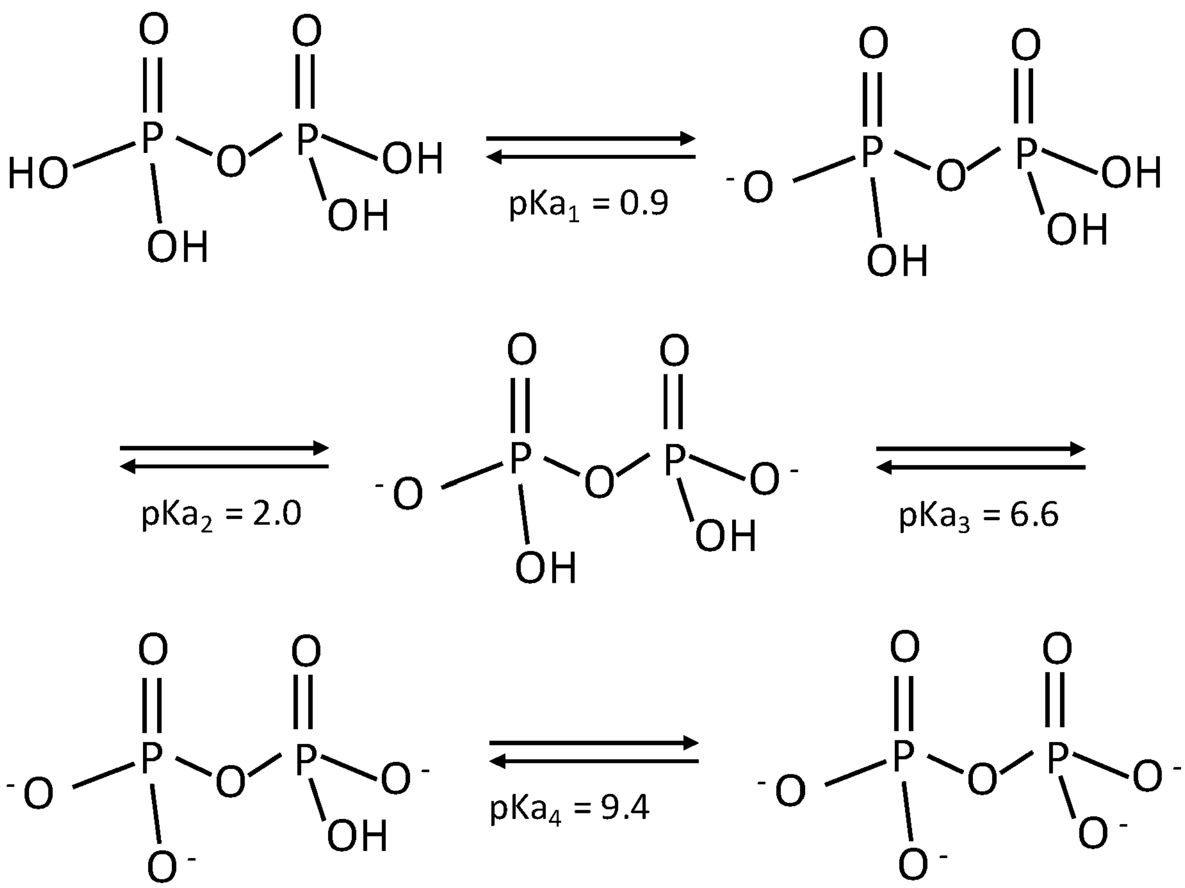

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H067060 U
- JP 2827649 B
- EP 3450972 A1
- JP H06331629 A